(19) Europäisches Patentamt
European Patent Office
Office européen des brevets

(11) **EP 4 636 775 A1**

(12) **EUROPEAN PATENT APPLICATION**
published in accordance with Art. 153(4) EPC

(43) Date of publication:
**22.10.2025 Bulletin 2025/43**

(51) International Patent Classification (IPC):
*G16C 60/00* (2019.01)     *G16C 20/20* (2019.01)
*G06F 30/28* (2020.01)     *H01M 4/133* (2010.01)

(21) Application number: 25732869.0

(86) International application number:
**PCT/CN2025/079025**

(22) Date of filing: **25.02.2025**

(87) International publication number:
**WO 2025/131141 (26.06.2025 Gazette 2025/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.05.2024  CN 202410592927**

(71) Applicant: Eve Energy Co., Ltd.
Huizhou, Guangdong 516006 (CN)

(72) Inventor: LIN, Guanzuo
Huizhou, Guangdong 516006 (CN)

(74) Representative: Regimbeau
20, rue de Chazelles
75847 Paris Cedex 17 (FR)

(54) **MATERIAL PROPORTION DETERMINATION METHOD FOR CONDUCTIVE MATERIAL IN BATTERY CONDUCTIVE FILM, AND ELECTRONIC DEVICE AND STORAGE MEDIUM**

(57)    Provided are a method for determining a material proportion of a conductive material in a battery conductive sheet, an electronic device, and a storage medium. The method for determining a material proportion of a conductive material in a battery conductive sheet includes determining (S110) a percolation curve model of a preset conductive sheet, where the preset conductive sheet includes at least one conductive material, and the percolation curve model is a relation curve between the preset resistance value of the preset conductive sheet and the material proportion of each conductive material in the preset conductive sheet; and determining (S120) the material proportion of each conductive material in a target conductive sheet based on the target resistance value of the target conductive sheet and the percolation curve model. This enables faster and more accurate determination of the material proportion of each conductive material in the target conductive sheet, thereby reducing both the time cost and the production cost of the target conductive sheet.

Determine a percolation curve model of a preset conductive sheet, where the preset conductive sheet includes at least one conductive material, and the percolation curve model is a relation curve between the preset resistance value of the preset conductive sheet and the material proportion of each conductive material in the preset conductive sheet — S110

Determine the material proportion of each conductive material in a target conductive sheet based on the target resistance value of the target conductive sheet and the percolation curve model — S120

**FIG. 1**

EP 4 636 775 A1

## Description

This application claims priority to Chinese Patent Application No. 202410592927.2 filed with the China National Intellectual Property Administration (CNIPA) on May 13, 2024, the disclosure of which is incorporated herein by reference in its entirety.

TECHNICAL FIELD

This application relates to the field of conductive material proportioning, for example, a method for determining a material proportion of a conductive material in a battery conductive sheet, an electronic device, and a storage medium.

BACKGROUND

With the continuously increasing market demand for the overall performance of batteries, the traditional positive-electrode conductive agents with conductive carbon black (super P, SP) can no longer meet the requirements for high energy density and high-power discharge, and future conductive agent systems will be diversified composite systems.
In recent years, with the development of high-voltage systems, each battery manufacturer has been developing positive-electrode conductive agent formulations of different types and proportions.

TECHNICAL PROBLEM

The positive-electrode conductive agent formulations developed by different battery manufacturers vary. There is no clearly defined solution path in terms of positive-electrode conductive agent formulations in the industry. Moreover, the determination of the material proportion of each conductive material in a conductive agent is relatively complex.

TECHNICAL SOLUTION

According to an aspect of this application, a method for determining a material proportion of a conductive material in a battery conductive sheet is provided. The method for determining the material proportion of the conductive material in the battery conductive sheet includes the following:
A percolation curve model of a preset conductive sheet is determined, where the preset conductive sheet includes at least one conductive material, and the percolation curve model is a relation curve between the preset resistance value of the preset conductive sheet and the material proportion of each conductive material in the preset conductive sheet.
The material proportion of each conductive material in a target conductive sheet is determined based on the target resistance value of the target conductive sheet and the percolation curve model.
In some embodiments, determining the percolation curve model of the preset conductive sheet includes the following:

acquiring multiple conductive agent formulations and determining the preset resistance value of a preset conductive sheet corresponding to each conductive agent formulation of the conductive agent formulations based on each conductive agent formulation;

setting the initial conductive weight value of each conductive material and determining an initial percolation curve model based on the initial conductive weight value of each conductive material, the material proportion of each conductive material in each conductive agent formulation, and the preset resistance value of the preset conductive sheet corresponding to each conductive agent formulation; and

adjusting the initial conductive weight value of each conductive material in the initial percolation curve model to determine the percolation curve model.

In some embodiments, adjusting the initial conductive weight value of each conductive material in the initial percolation curve model to determine the percolation curve model includes the following:

determining goodness of fit of the initial percolation curve model based on the initial percolation curve model;

adjusting the initial conductive weight value based on the goodness of fit of the initial percolation curve model to make the goodness of fit equal to the best goodness of fit; and

determining the percolation curve model based on a conductive weight value corresponding to the best goodness of fit.

In some embodiments, the percolation curve model satisfies:

$$y = \alpha(ax_1 + bx_2 + \ldots + cx_n)^{\beta}.$$

Here y is the resistance value of a conductive sheet, *a* is a conductive weight value corresponding to a first conductive material, *b* is a conductive weight value corresponding to a second conductive material, c is a conductive weight value corresponding to an n-th conductive material, $\alpha$ is a first fitting parameter, $\beta$ is a second fitting parameter, $x_1$ is the material proportion of the first conductive material, $x_2$ is the material proportion of the second conductive material, and $x_n$ is the material proportion of the n-th conductive material.

In some embodiments, the preset resistance value of the preset conductive sheet includes a first sheet resistance value after coating or a second sheet resistance value after cold pressing.

In some embodiments, the conductive material includes at least one of a single-walled carbon nanotube conductive material, a multi-walled carbon nanotube conductive material, or a carbon black conductive material.

The material proportion of the single-walled carbon nanotube conductive material is 0 to 0.12.

The material proportion of the multi-walled carbon nanotube conductive material is 0 to 1.05.

The material proportion of the carbon black conductive material is 0 to 2.

According to another aspect of this application, an apparatus for determining a material proportion of a conductive material in a battery conductive sheet is provided. The apparatus for determining a material proportion of a conductive material in a battery conductive sheet includes a percolation curve determination module and a material proportion determination module.

The percolation curve determination module is configured to determine a percolation curve model of a preset conductive sheet, where the preset conductive sheet includes at least two conductive materials, and the percolation curve model is a relation curve between the preset resistance value of the preset conductive sheet and the material proportion of each conductive material in the preset conductive sheet.

The material proportion determination module is configured to determine the material proportion of each conductive material in a target conductive sheet based on the target resistance value of the target conductive sheet and the percolation curve model.

In some embodiments, the percolation curve determination module includes a sheet resistance determination unit, an initial curve determination unit, and a percolation curve determination unit.

The sheet resistance determination unit is configured to acquire multiple conductive agent formulations and determine the preset resistance value of a preset conductive sheet corresponding to each conductive agent formulation based on each conductive agent formulation.

The initial curve determination unit is configured to set an initial conductive weight value of each conductive material and determine an initial percolation curve model based on the initial conductive weight value of each conductive material, the material proportion of each conductive material in each conductive agent formulation, and the preset resistance value of the preset conductive sheet corresponding to each conductive agent formulation.

The percolation curve determination unit is configured to adjust the initial conductive weight value of each conductive material in the initial percolation curve model to determine the percolation curve model.

According to another aspect of this application, an electronic device is provided.

The electronic device includes at least one processor; and a memory communicatively connected to the at least one processor.

The memory stores a computer program executable by the at least one processor to enable the at least one processor to perform the method for determining a material proportion of a conductive material in a battery conductive sheet according to any embodiment of this application.

According to another aspect of this application, a computer-readable storage medium storing computer instructions which, when executed by a processor, cause the processor to perform the method for determining a material proportion of a conductive material in a battery conductive sheet according to any embodiment of this application.

BENEFICIAL EFFECTS

The method for determining a material proportion of a conductive material in a battery conductive sheet according to this application includes determining a percolation curve model of a preset conductive sheet and determining the material proportion of each conductive material in a target conductive sheet based on the percolation curve model and the desired conductivity of the target conductive sheet, that is, the target resistance value of the target conductive sheet. This enables faster and more accurate determination of the material proportion of each conductive material in the target conductive sheet, thereby reducing both the time cost and the production cost of the conductive sheet.

BRIEF DESCRIPTION OF THE DRAWINGS

FIG. 1 is a flowchart of a method for determining a material proportion of a conductive material in a battery conductive sheet according to embodiments of this application.

FIG. 2 is another flowchart of a method for determining a material proportion of a conductive material in a battery conductive sheet according to embodiments of this application.

FIG. 3 is a diagram of a coated conductive percolation curve model according to embodiments of this application.

FIG. 4 is a diagram of a cold-pressed conductive percolation curve model according to embodiments of this application.

FIG. 5 is a diagram illustrating the structure of an apparatus for determining a material proportion of a conductive material in a battery conductive sheet according to embodiments of this application.

FIG. 6 is a diagram illustrating the structure of an electronic device for implementing embodiments of this application.

DETAILED DESCRIPTION

It is to be noted that terms such as "first" and "second" in the description, claims, and drawings of this application are used to distinguish between similar objects and are not necessarily used to describe a particular order or sequence. It is to be understood that data used in this manner are interchangeable where appropriate so that the embodiments of this application described herein can be implemented in an order not illustrated or described herein. Additionally, terms "including" and "having" and any variations thereof are intended to encompass a non-exclusive inclusion. For example, this application not only includes a process, method, system, product or device in a series of steps or units listed in embodiments of this application but may also include a series of steps or units that are not expressly listed in embodiments of this application or a system, a product, a device, or a process or method therein.

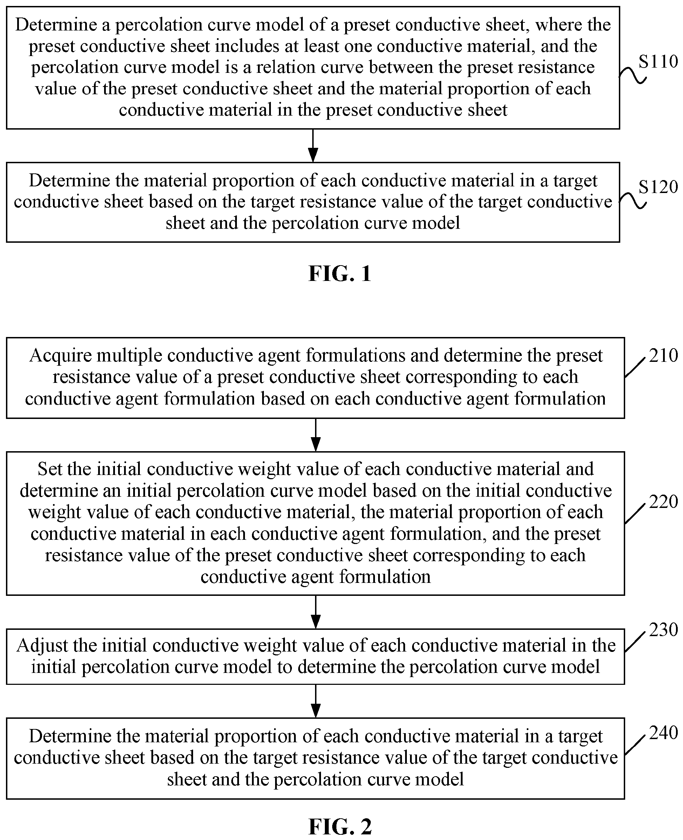

An embodiment of this application provides a method for determining a material proportion of a conductive material in a battery conductive sheet. FIG. 1 is a flowchart of a method for determining a material proportion of a conductive material in a battery conductive sheet according to an embodiment of this application. Referring to FIG. 1, the method for determining the material proportion of the conductive material in the battery conductive sheet includes the following:

In S110, a percolation curve model of a preset conductive sheet is determined, where the preset conductive sheet includes at least one conductive material, and the percolation curve model is a relation curve between the preset resistance value of the preset conductive sheet and the material proportion of each conductive material in the preset conductive sheet.

The term "at least one" means "one or more" and is not limited in this embodiment of this application. By way of example, the conductive sheet may include one or more of the following conductive materials: single-walled carbon nanotube (SWCNT) conductive material, multi-walled carbon nanotube (MWCNT) conductive material, array conductive material, conductive graphite, graphene, and carbon black conductive material. The carbon black conductive material includes super P material, acetylene black material, and Ketjen black material. The method for determining a material proportion of a conductive material in a battery conductive sheet according to this embodiment of this application is applicable to fields such as lithium-ion batteries, solar cells, and conductive ceramics.

By way of example, it is feasible to acquire multiple conductive agent formulations and determine the preset resistance value of a preset conductive sheet corresponding to each conductive agent formulation based on each conductive agent formulation; set the initial conductive weight value of each conductive material and determine an initial percolation curve model based on the initial conductive weight value of each conductive material, the material proportion of each conductive material in each conductive agent formulation, and the preset resistance value of the preset conductive sheet corresponding to each conductive agent formulation; and adjust the initial conductive weight value of each conductive material in the initial percolation curve model to determine the percolation curve model.

In S120, the material proportion of each conductive material in a target conductive sheet is determined based on the target resistance value of the target conductive sheet and the percolation curve model.

By way of example, it is feasible to determine the material proportion of each conductive material in the target conductive sheet through the percolation curve model based on the target resistance value of the target conductive sheet and practical application requirements, such as processing performance requirements, electrical performance requirements, and cost requirements. By way of example, it is feasible to determine the optimal added amount of each conductive material with the assistance of the inflection point of the percolation curve model, that is, the percolation threshold, thereby reducing the costs and achieving superior conductivity. When the production cost is a concern, it is also feasible to select more cost-effective conductive materials based on the percolation curve model while ensuring the conductivity of the target conductive sheet.

The method for determining a material proportion of a conductive material in a battery conductive sheet according to this embodiment of this application includes determining a percolation curve model of a preset conductive sheet and determining the material proportion of each conductive material in a target conductive sheet based on the percolation curve model and the desired conductivity of the target conductive sheet, that is, the target resistance value of the target conductive sheet. This embodiment of this application can determine the material proportion of each conductive material in the target conductive sheet based on the percolation curve model of the preset conductive sheet and the target resistance value of the target conductive sheet. This enables faster and more accurate determination of the material proportion of each conductive material in the target conductive sheet, thereby saving both the time cost and the production cost of the conductive sheet.

FIG. 2 is another flowchart of a method for determining a material proportion of a conductive material in a battery conductive sheet according to an embodiment of this application. As shown in FIG. 2, the method includes the following: In S210, multiple conductive agent formulations are acquired, and the preset resistance value of a preset conductive sheet corresponding to each conductive agent formulation is determined based on each conductive agent formulation.

By way of example, it is feasible to design different conductive agent formulations and measure the preset resistance value of the corresponding preset conductive sheet based on each conductive agent formulation or to extract different conductive agent formulations based on existing historical data and determine the preset resistance value of the preset conductive sheet corresponding to each conductive agent formulation.

In S220, an initial conductive weight value of each conductive material is set, and an initial percolation curve model is determined based on the initial conductive weight value of each conductive material, the material proportion of each conductive material in each conductive agent formulation, and the preset resistance value of the preset conductive sheet corresponding to each conductive agent formulation.

By way of example, the percolation curve model may be as follows:

$$y = \alpha(ax_1 + bx_2 + ... + cx_n)^\beta$$

Here y is the resistance value of a conductive sheet, *a* is a conductive weight value corresponding to a first conductive material, *b* is a conductive weight value corresponding to a second conductive material, *c* is a conductive weight value corresponding to an n-th conductive material, $\alpha$ is a first fitting parameter, $\beta$ is a second fitting parameter, $x_1$ is the material proportion of the first conductive material, $x_2$ is the material proportion of the second conductive material, and $x_n$ is the material proportion of the n-th conductive material.

The first fitting parameter $\alpha$ and the second fitting parameter $\beta$ in the percolation curve model may be determined based on the initial conductive weight value of each conductive material, that is, the values of *a, b, ..., c* in the percolation curve model, based on the material proportion of each conductive material in each conductive agent formula, that is, the values of $x_1$, $x_2$, ..., $x_n$ in the percolation curve model, and based on the preset resistance value of the preset conductive sheet corresponding to each conductive agent formula, that is, the value of y in the percolation curve model. Then the initial percolation curve model may be determined.

In S230, the initial conductive weight value of each conductive material in the initial percolation curve model is adjusted to determine the percolation curve model.

By way of example, it is feasible to determine the goodness of fit of the initial percolation curve model based on the initial percolation curve model; adjust the initial conductive weight value based on the goodness of fit of the initial percolation curve model to make the goodness of fit equal to the best goodness of fit; and determine the percolation curve model based on a conductive weight value corresponding to the best goodness of fit. The best goodness of fit may be set according to actual conditions. This is not limited here.

In S240, the material proportion of each conductive material in a target conductive sheet is determined based on the target resistance value of the target conductive sheet and the percolation curve model.

For the operations and effects of S240, reference is made to S120. The details are not described here.

In the method for determining a material proportion of a conductive material in a battery conductive sheet according to this embodiment of this application, multiple conductive agent formulations are acquired, and the preset resistance value of a preset conductive sheet corresponding to each conductive agent formulation is determined based on each conductive agent formulation; the initial conductive weight value of each conductive material is set, and an initial percolation curve model is determined based on the initial conductive weight value of each conductive material, the material proportion of each conductive material in each conductive agent formulation, and the preset resistance value of the preset conductive sheet corresponding to each conductive agent formulation; the initial conductive weight value of each conductive material in the initial percolation curve model is adjusted to determine the percolation curve model; and the material proportion of each conductive material in a target conductive sheet is determined based on the target resistance value of the target conductive sheet and the percolation curve model. This embodiment of this application can determine the material proportion of each conductive material in the target conductive sheet based on the percolation curve model of the preset conductive sheet and the target resistance value of the target conductive sheet. This enables faster and more accurate determination of the material proportion of each conductive material in the target conductive sheet, thereby reducing both the time cost and the production cost of the conductive sheet.

In an embodiment, adjusting the initial conductive weight value of each conductive material in the initial percolation curve model to determine the percolation curve model includes the following:
The goodness of fit of the initial percolation curve model is determined based on the initial percolation curve model.

The initial conductive weight value of each conductive material is adjusted based on the goodness of fit of the initial percolation curve model to make the goodness of fit equal to the best goodness of fit.

The percolation curve model is determined based on a conductive weight value corresponding to the best goodness of fit.

By way of example, it is feasible to determine the goodness of fit of the initial percolation curve model based on the initial percolation curve model and determine whether the current goodness of fit is equal to the best goodness of fit; if the current goodness of fit is equal to the best goodness of fit, the percolation curve model is determined based on the conductive weight value corresponding to the best goodness of fit; if the current goodness of fit is not equal to the best goodness of fit, the initial conductive weight value of each conductive material is adjusted based on the goodness of fit of the initial percolation curve model, a new percolation curve model is determined based on the adjusted conductive weight value, the new percolation curve model is fitted to determine the goodness of fit of the new percolation curve model, and it is determined whether the current goodness of fit is equal to the best goodness of fit; if the current goodness of fit is equal to the best goodness of fit, the percolation curve model is determined based on the conductive weight value corresponding to the best goodness of fit; and if the current goodness of fit is not equal to the best goodness of fit, it is continued adjusting the conductive weight value of each conductive material based on the goodness of fit of the current percolation curve model until the goodness of fit of the percolation curve model is equal to the best goodness of fit, and the percolation curve model is determined based on the conductive weight value corresponding to the best goodness of fit.

In an embodiment, the percolation curve model satisfies:

$$y = \alpha(ax_1 + bx_2 + \ldots + cx_n)^\beta$$

Here y is the resistance value of a conductive sheet, $a$ is a conductive weight value corresponding to a first conductive material, $b$ is a conductive weight value corresponding to a second conductive material, $c$ is a conductive weight value corresponding to an n-th conductive material, $\alpha$ is a first fitting parameter, $\beta$ is a second fitting parameter, $x_1$ is the material proportion of the first conductive material, $x_2$ is the material proportion of the second conductive material, and $x_n$ is the material proportion of the n-th conductive material.

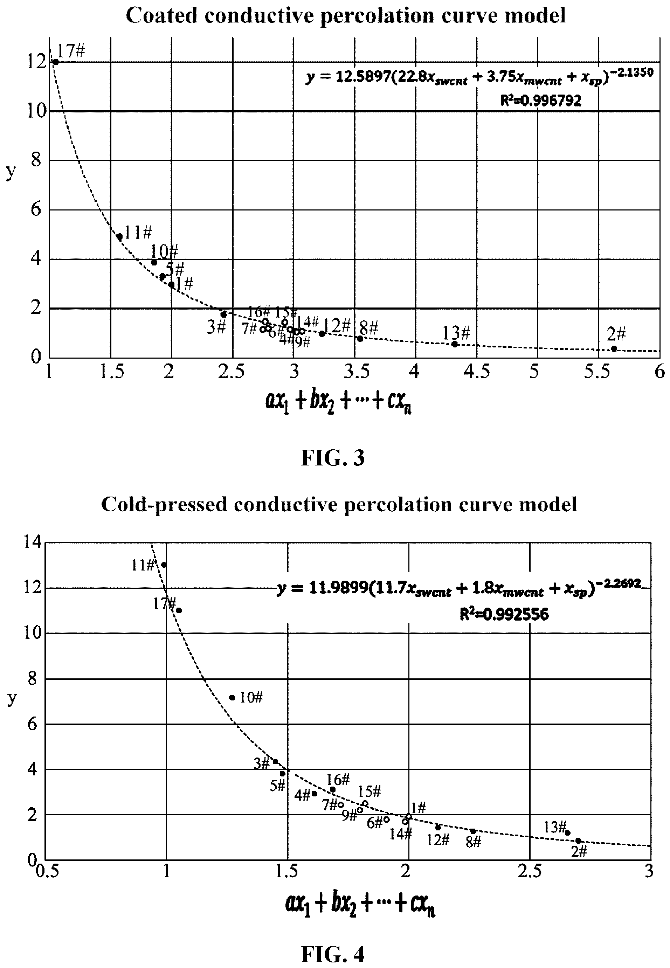

After coating, it is feasible to measure the preset resistance value of the coated preset conductive sheet and determine the coated conductive percolation curve model by using the method for determining the percolation curve model. FIG. 3 is a diagram of a coated conductive percolation curve model according to embodiments of this application. By way of example, referring to FIG. 3, the coated conductive percolation curve model may be determined through the 17 data points as shown in FIG. 3. After cold pressing, the preset resistance value of the cold-pressed preset conductive sheet may be measured and the cold-pressed conductive percolation curve model may be determined by using the method for determining the percolation curve model. FIG. 4 is a diagram of a cold-pressed conductive percolation curve model according to embodiments of this application. By way of example, referring to FIG. 4, the cold-pressed conductive percolation curve model may be determined through the 17 data points as shown in FIG. 4.

In an embodiment, the preset resistance value of the preset conductive sheet includes a first sheet resistance value after coating or a second sheet resistance value after cold pressing.

In an embodiment, the conductive material includes at least one of a single-walled carbon nanotube conductive material, a multi-walled carbon nanotube conductive material, or a carbon black conductive material.

The material proportion of the single-walled carbon nanotube conductive material is 0 to 0.12.

The material proportion of the multi-walled carbon nanotube conductive material is 0 to 1.05.

## EP 4 636 775 A1

The material proportion of the carbon black conductive material is 0 to 2.

By way of example, the embodiments of this application provide a positive-electrode conductive agent formulation based on at least one of the single-walled carbon nanotube conductive material, the multi-walled carbon nanotube conductive material, or the carbon black conductive material. Table 1 describes formulation proportions of a positive electrode tab after coating. Table 2 describes formulation proportions of a positive electrode tab after cold pressing. The following table describes the formulation composition of the positive electrode tab.

Table 1 Formulation proportions of a positive electrode tab after coating

| Slurry Formulati on | LCO | SWCN T | MWC NT | SP | PVDF | Total Conductive Percolation Weight Value | Coated Sheet Resistan ce | Calculat ed Value |
|---|---|---|---|---|---|---|---|---|
| Scheme 1# | 97 | 0 | 0 | 2 | 1 | 2 | 2.985 | 2.866 |
| Scheme 2# | 97.5 | 0 | 1.5 | 0 | 1 | 5.625 | 0.396 | 0.315 |
| Scheme 3# | 98 | 0 | 0.5 | 0.55 | 0.95 | 2.425 | 1.760 | 1.900 |
| Scheme 4# | 98 | 0 | 0.7 | 0.35 | 0.95 | 2.975 | 1.144 | 1.228 |
| Scheme 5# | 98 | 0.04 | 0 | 1.01 | 0.95 | 1.922 | 3.311 | 3.120 |
| Scheme 6# | 98 | 0.08 | 0 | 0.97 | 0.95 | 2.794 | 1.199 | 1.404 |
| Scheme 7# | 98 | 0.04 | 0.3 | 0.71 | 0.95 | 2.747 | 1.159 | 1.456 |
| Scheme 8# | 98 | 0.08 | 0.2 | 0.97 | 0.95 | 3.544 | 0.798 | 0.845 |
| Scheme 9# | 98 | 0.04 | 0.4 | 0.61 | 0.95 | 3.022 | 1.068 | 1.187 |
| Scheme 10# | 98 | 0 | 0.3 | 0.73 | 0.97 | 1.855 | 3.871 | 3.366 |
| Scheme 11# | 98 | 0 | 0.3 | 0.45 | 1.25 | 1.575 | 4.929 | 4.773 |
| Scheme 12# | 98 | 0.1 | 0 | 0.95 | 0.95 | 3.23 | 0.997 | 1.030 |
| Scheme 13# | 98 | 0.15 | 0 | 0.9 | 0.95 | 4.32 | 0.584 | 0.554 |
| Scheme 14# | 98 | 0.08 | 0.1 | 0.87 | 0.95 | 3.069 | 1.090 | 1.149 |
| Scheme 15# | 98 | 0.1 | 0 | 0.65 | 1.25 | 2.93 | 1.448 | 1.268 |
| Scheme 16# | 98 | 0.08 | 0.1 | 0.57 | 1.25 | 2.769 | 1.485 | 1.431 |
| Scheme 17# | 98 | 0 | 0 | 1.05 | 0.95 | 1.05 | 12.016 | 11.344 |
| Weight Proportio n of Coated Conducti ve Agent | | 22.8 | 3.75 | 1 | | | | |
| Coating Substituti on Proportio n | | 1 | 6.08 | 22.8 | | | | |

Table 2 Formulation proportions of a positive electrode tab after cold pressing

| Slurry Formulati on | LCO | SWCN T | MWCN T | SP | PVDF | Total Conductive Percolation Weight Value | Cold-press ed Sheet Resistance | Calculat ed Value |
|---|---|---|---|---|---|---|---|---|
| Scheme 1# | 97 | 0 | 0 | 2 | 1 | 2 | 1.920 | 1.855 |
| Scheme 2# | 97.5 | 0 | 1.5 | 0 | 1 | 2.7 | 0.875 | 0.827 |
| Scheme 3# | 98 | 0 | 0.5 | 0.55 | 0.95 | 1.45 | 4.348 | 4.409 |
| Scheme 4# | 98 | 0 | 0.7 | 0.35 | 0.95 | 1.61 | 2.947 | 3.326 |
| Scheme 5# | 98 | 0.04 | 0 | 1.01 | 0.95 | 1.478 | 3.829 | 4.188 |
| Scheme 6# | 98 | 0.08 | 0 | 0.97 | 0.95 | 1.906 | 1.821 | 2.112 |

(continued)

| Slurry Formulation | LCO | SWCNT | MWCNT | SP | PVDF | Total Conductive Percolation Weight Value | Cold-pressed Sheet Resistance | Calculated Value |
|---|---|---|---|---|---|---|---|---|
| Scheme 7# | 98 | 0.04 | 0.3 | 0.71 | 0.95 | 1.718 | 2.448 | 2.793 |
| Scheme 8# | 98 | 0.08 | 0.2 | 0.97 | 0.95 | 2.266 | 1.276 | 1.325 |
| Scheme 9# | 98 | 0.04 | 0.4 | 0.61 | 0.95 | 1.798 | 2.225 | 2.471 |
| Scheme 10# | 98 | 0 | 0.3 | 0.73 | 0.97 | 1.27 | 7.176 | 6.300 |
| Scheme 11# | 98 | 0 | 0.3 | 0.45 | 1.25 | 0.99 | 13.012 | 12.319 |
| Scheme 12# | 98 | 0.1 | 0 | 0.95 | 0.95 | 2.12 | 1.433 | 1.586 |
| Scheme 13# | 98 | 0.15 | 0 | 0.9 | 0.95 | 2.655 | 1.213 | 0.865 |
| Scheme 14# | 98 | 0.08 | 0.1 | 0.87 | 0.95 | 1.986 | 1.697 | 1.890 |
| Scheme 15# | 98 | 0.1 | 0 | 0.65 | 1.25 | 1.82 | 2.518 | 2.391 |
| Scheme 16# | 98 | 0.08 | 0.1 | 0.57 | 1.25 | 1.686 | 3.126 | 2.938 |
| Scheme 17# | 98 | 0 | 0 | 1.05 | 0.95 | 1.05 | 11.012 | 10.514 |
| Weight Proportion of Cold-pressed Conductive Agent | | 11.7 | 1.8 | 1 | | | | |

The preparation method of a positive electrode tab includes the following: preparing polyvinylidene fluoride (PVDF) glue; stirring the PVDF glue, a positive electrode main material, and a composite conductive agent to obtain a mixture slurry, where the positive electrode main material is lithium cobalt oxide (LCO) and the PVDF glue is polyvinylidene fluoride glue; coating the slurry on an aluminum foil current collector and measuring the resistance of the coated conductive sheet; and cold-pressing the coated conductive sheet and measuring the resistance of the cold-pressed conductive sheet. The total conductive percolation weight value in the formulation proportion table is the calculated result of "$ax_1 + bx_2 + ... + cx_n$" in the percolation curve model.

After determining the resistance of the coated conductive sheet, the coated conductive percolation curve model may be determined based on values in the formulation proportion table of the positive electrode tab after coating by using the percolation curve determination method. By way of example, the curve shown in FIG. 3 is represented as follows:

$$y = 12.5897(22.8x_{swcnt} + 3.75x_{mwcnt} + x_{sp})^{-2.1350}$$

Based on the conductive weight value of SWCNT, the conductive weight value of MWCNT, and the conductive weight value of SP in the percolation curve, the substitution ratio of SWCNT, MWCNT, and SP is 1:6.08:22.8. It can be seen from Table 1 that the resistance value of the coated sheet calculated using the percolation curve model shown in FIG. 3 is close to the measured resistance value of the coated sheet, indicating a good fit of the coated percolation curve model. Therefore, the material proportion of each conductive material in the conductive sheet can be determined using the coated conductive percolation curve model shown in FIG. 3. Based on the inflection point of the coated percolation curve, it can be determined that schemes 4, 6, 7, 9, 14, 15, and 16 reach the percolation threshold. The appropriate scheme may be selected based on the requirements for electrical performance and production cost. After determining the resistance of the cold-pressed conductive sheet, it is feasible to determine the cold-pressed conductive percolation curve model by using the percolation curve determination method. By way of example, the curve shown in FIG. 4 is represented as:

$$y = 11.9899(11.7x_{swcnt} + 1.8x_{mwcnt} + x_{sp})^{-2.2692}$$

Based on the conductive weight value of SWCNT, the conductive weight value of MWCNT, and the conductive weight value of SP in the percolation curve, the substitution ratio of SWCNT, MWCNT, and SP is 1:6.5:11.7. It can be seen from Table 2 that the resistance value of the cold-pressed sheet calculated using the percolation curve model shown in FIG. 4 is close to the measured resistance value of the cold-pressed sheet, indicating a good fit of the cold-pressed percolation curve model. Therefore, the material proportion of each conductive material in the conductive sheet can be determined using the cold-pressed conductive percolation curve model shown in FIG. 4. Based on the inflection point of the cold-pressed percolation curve, it can be determined that schemes 1, 6, 8, 9, 12, 14, and 15 reach the percolation threshold. The appropriate scheme may be selected based on the requirements for electrical performance and production cost. Based on the coated percolation curve and the cold-pressed percolation curve, it can be seen that schemes 6, 9, 14, and 15 exhibit superior conductivity. The scheme meeting practical requirements may be selected based on the requirements for electrical performance and production cost.

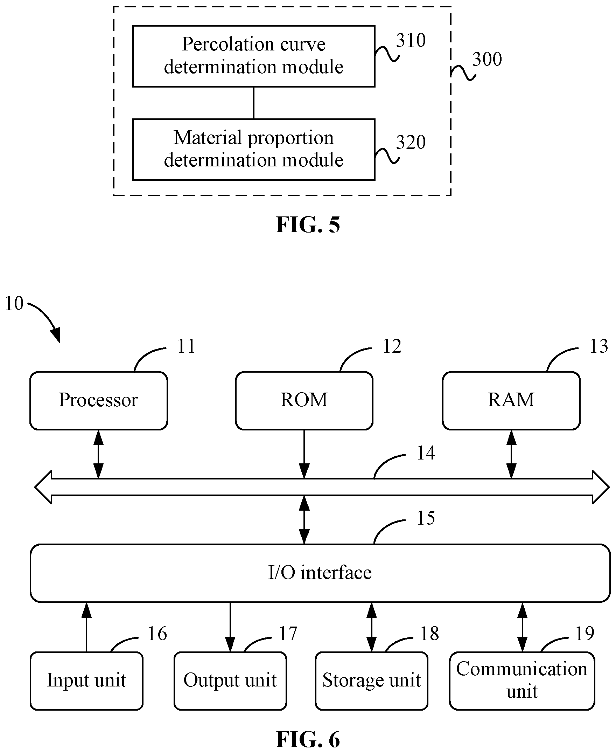

An embodiment of this application provides an apparatus for determining a material proportion of a conductive material in a battery conductive sheet. FIG. 5 is a diagram illustrating the structure of an apparatus for determining a material proportion of a conductive material in a battery conductive sheet according to an embodiment of this application. Referring to FIG. 5, the apparatus 300 for determining a material proportion of a conductive material in a battery conductive sheet includes a percolation curve determination module 310 and a material proportion determination module 320.

The percolation curve determination module 310 is configured to determine a percolation curve model of a preset conductive sheet, where the preset conductive sheet includes at least one conductive material, and the percolation curve model is a relation curve between the preset resistance value of the preset conductive sheet and the material proportion of each conductive material in the preset conductive sheet.

The material proportion determination module 320 is configured to determine the material proportion of each conductive material in a target conductive sheet based on the target resistance value of the target conductive sheet and the percolation curve model.

In an embodiment, the percolation curve determination module 310 includes a sheet resistance determination unit, an initial curve determination unit, and a percolation curve determination unit.

The sheet resistance determination unit is configured to acquire multiple conductive agent formulations and determine the preset resistance value of a preset conductive sheet corresponding to each conductive agent formulation based on each conductive agent formulation.

The initial curve determination unit is configured to set the initial conductive weight value of each conductive material and determine an initial percolation curve model based on the initial conductive weight value of each conductive material, the material proportion of each conductive material in each conductive agent formulation, and the preset resistance value of the preset conductive sheet corresponding to each conductive agent formulation.

The percolation curve determination unit is configured to adjust the initial conductive weight value of each conductive material in the initial percolation curve model to determine the percolation curve model.

In an embodiment, the percolation curve determination module includes a fit goodness determination subunit, a best fit goodness determination subunit, and a percolation curve determination subunit.

The fit goodness determination subunit is configured to determine goodness of fit of the initial percolation curve model based on the initial percolation curve model.

The best fit goodness determination subunit is configured to adjust the initial conductive weight value based on the goodness of fit of the initial percolation curve model to make the goodness of fit equal to the best goodness of fit.

The percolation curve determination subunit is configured to determine the percolation curve model based on a conductive weight value corresponding to the best goodness of fit.

In an embodiment, the percolation curve model satisfies:

$$y = \alpha(ax_1 + bx_2 + \ldots + cx_n)^\beta$$

Here y is the resistance value of a conductive sheet, $a$ is a conductive weight value corresponding to a first conductive material, $b$ is a conductive weight value corresponding to a second conductive material, $c$ is a conductive weight value corresponding to an n-th conductive material, $\alpha$ is a first fitting parameter, $\beta$ is a second fitting parameter, $x_1$ is the material proportion of the first conductive material, $x_2$ is the material proportion of the second conductive material, and $x_n$ is the material proportion of the n-th conductive material.

In an embodiment, the preset resistance value of the preset conductive sheet includes a first sheet resistance value after coating or a second sheet resistance value after cold pressing.

In an embodiment, the conductive material includes at least one of a single-walled carbon nanotube conductive material, a multi-walled carbon nanotube conductive material, or a carbon black conductive material.

The material proportion of the single-walled carbon nanotube conductive material is 0 to 0.12.

The material proportion of the multi-walled carbon nanotube conductive material is 0 to 1.05.

The material proportion of the carbon black conductive material is 0 to 2.

The apparatus for determining the material proportion of the conductive material in the battery conductive sheet according to this embodiment of this application can perform the method for determining the material proportion of the conductive material in the battery conductive sheet according to any embodiment of this application and has function modules and beneficial effects corresponding to the performed method.

FIG. 6 is a diagram illustrating the structure of an electronic device for implementing embodiments of this application. The electronic device may include various forms of digital computers, for example, laptop computers, desktop computers, worktables, personal digital assistants, servers, blade servers, mainframe computers, and other applicable computers. The electronic device may also include various forms of mobile apparatuses, for example, a personal digital assistant, a cellphone, a smartphone, a wearable device (such as a helmet, glasses, or a watch), and another similar computing apparatus. Herein the shown components, the connections and relationships between these components, and the functions of these components are merely illustrative and are not intended to limit the implementation of this application as described and/or claimed herein.

As shown in FIG. 6, the electronic device 10 includes at least one processor 11 and a memory communicatively connected to the at least one processor 11, such as a read-only memory (ROM) 12 and a random-access memory (RAM) 13. The memory stores a computer program executable by the at least one processor. The at least one processor 11 can perform various appropriate actions and processing according to a computer program stored in the read-only memory (ROM) 12 or a computer program loaded into the random-access memory (RAM) 13 from a storage unit 18. The RAM 13 may also store various programs and data required for the operation of the electronic device 10. The at least one processor 11, the ROM 12, and the RAM 13 are connected to each other through a bus 14. An input/output (I/O) interface 15 is also connected to the bus 14.

Multiple components in the electronic device 10 are connected to the I/O interface 15. The multiple components include input units 16 such as a keyboard and a mouse, output units 17 such as various types of displays and speakers, the storage unit 18 such as a magnetic disk or an optical disk, and a communication unit 19 such as a network card, a modem, or a wireless communication transceiver. The communication unit 19 allows the electronic device 10 to exchange information/data with other devices over a computer network such as the Internet and/or various telecommunications networks.

The at least one processor 11 may be various general-purpose and/or special-purpose processing components having processing and computing capabilities. Examples of the processor 11 include, but are not limited to, a central processing unit (CPU), a graphics processing unit (GPU), a special-purpose artificial intelligence (AI) computing chip, a processor executing machine learning models and algorithms, a digital signal processing (DSP), and any appropriate processor, controller, or microcontroller. The at least one processor 11 performs various preceding methods and processing, such as the method for determining a material proportion of a conductive material in a battery conductive sheet.

In some embodiments, the method for determining the material proportion of the conductive material in the battery conductive sheet may be implemented as computer programs tangibly contained in a computer-readable storage medium such as the storage unit 18. In some embodiments, part or all of the computer programs may be loaded and/or installed onto the electronic device 10 via the ROM 12 and/or the communication unit 19. When the computer programs are loaded to the RAM 13 and executed by the at least one processor 11, one or more steps of the method for determining a material proportion of a conductive material in a battery conductive sheet may be performed. Alternatively, in other embodiments, the at least one processor 11 may be configured, in any other suitable manner (for example, by firmware), to perform the method for determining a material proportion of a conductive material in a battery conductive sheet.

The various embodiments of the systems and techniques described herein may be implemented in digital electronic circuitry, integrated circuitry, a field-programmable gate array (FPGA), an application-specific integrated circuit (ASIC), application specific standard product (ASSP), a system on a chip (SoC), a complex programmable logic device (CPLD), computer hardware, firmware, software, and/or a combination thereof. These embodiments may include implementations in one or more computer programs. The one or more computer programs are executable and/or interpretable on a programmable system including at least one programmable processor. The at least one programmable processor may be a special-purpose or general-purpose programmable processor for receiving data and instructions from a storage system, at least one input apparatus and at least one output apparatus and transmitting data and instructions to the storage system, the at least one input apparatus and the at least one output apparatus.

Computer programs for implementation of the methods of this application may be written in one programming language or any combination of multiple programming languages. The computer programs may be provided for a processor of a general-purpose computer, a special-purpose computer, or another programmable data processing apparatus to enable functions/operations specified in a flowchart and/or a block diagram to be implemented when the computer programs are executed by the processor. The computer programs may be executed entirely on a machine, partly on a machine, as a stand-alone software package, partly on a machine and partly on a remote machine, or entirely on a remote machine or a server.

In the context of this application, the computer-readable storage medium may be a tangible medium that may include or store a computer program for use by or in connection with an instruction execution system, apparatus, or device. The computer-readable storage medium may include, but is not limited to, an electronic, magnetic, optical, electromagnetic, infrared, or semiconductor system, apparatus, or device, or any appropriate combination thereof. Alternatively, the computer-readable storage medium may be a machine-readable signal medium. The machine-readable storage medium may include an electrical connection based on one or more wires, a portable computer disk, a hard disk, a random-access memory (RAM), a read-only memory (ROM), an erasable programmable read-only memory (EPROM), a flash memory, an optical fiber, a portable compact disk read-only memory (CD-ROM), an optical memory device, a magnetic memory device or any suitable combination thereof.

In order that interaction with a user is provided, the systems and techniques described herein may be implemented in the electronic device. The electronic device has a display apparatus (for example, a cathode-ray tube (CRT) or a liquid-crystal display (LCD) monitor) for displaying information to the user; and a keyboard and a pointing apparatus (for example, a mouse or a trackball) through which the user can provide input for the electronic device. Other types of apparatuses may also be used for providing interaction with the user. For example, feedback provided for the user may be sensory feedback in any form (for example, visual feedback, auditory feedback, or haptic feedback). Moreover, input from the user may be received in any form (including acoustic input, voice input, or haptic input).

The systems and techniques described herein may be implemented in a computing system including a back-end component (for example, a data server), a computing system including a middleware component (for example, an application server), a computing system including a front-end component (for example, a client computer having a graphical user interface or a web browser through which the user can interact with embodiments of the systems and techniques described herein), or a computing system including any combination of such back-end, middleware, or front-end components. Components of a system may be interconnected by any form or medium of digital data communication (for example, a communication network). Examples of the communication network include a local area network (LAN), a wide area network (WAN), a blockchain network, and the Internet.

The computing system may include clients and servers. A client and a server are generally remote from each other and typically interact through a communication network. The relationship between the client and the server arises by virtue of computer programs running on respective computers and having a client-server relationship. The server may be a cloud server, also referred to as a cloud computing server or a cloud host. As a host product in a cloud computing service system, the server solves the defects of difficult management and weak service scalability in conventional physical host and virtual private server (VPS) services.

It is to be understood that various forms of the preceding flows may be used with steps reordered, added, or removed. For example, the steps described in this application may be executed in parallel, in sequence, or in a different order as long as the desired results of the technical solutions in this application are achieved. The execution sequence of these steps is not limited herein.

**Claims**

1. A method for determining a material proportion of a conductive material in a battery conductive sheet, comprising:

   determining a percolation curve model of a preset conductive sheet, wherein the preset conductive sheet comprises at least one conductive material, and the percolation curve model is a relation curve between a preset resistance value of the preset conductive sheet and a material proportion of each conductive material of the at least one conductive material in the preset conductive sheet; and
   determining a material proportion of each conductive material in a target conductive sheet based on a target resistance value of the target conductive sheet and the percolation curve model.

2. The method for determining the material proportion of the conductive material in the battery conductive sheet according to claim 1, wherein determining the percolation curve model of the preset conductive sheet comprises:

   acquiring a plurality of conductive agent formulations and determining a preset resistance value of a preset conductive sheet corresponding to each conductive agent formulation of the plurality of conductive agent formulations based on each conductive agent formulation;
   setting an initial conductive weight value of each conductive material and determining an initial percolation curve model based on the initial conductive weight value of each conductive material, a material proportion of each conductive material in each conductive agent formulation, and the preset resistance value of the preset conductive sheet corresponding to each conductive agent formulation; and
   adjusting the initial conductive weight value of each conductive material in the initial percolation curve model to determine the percolation curve model.

3. The method for determining the material proportion of the conductive material in the battery conductive sheet according to claim 2, wherein adjusting the initial conductive weight value of each conductive material in the initial percolation curve model to determine the percolation curve model comprises:

determining goodness of fit of the initial percolation curve model based on the initial percolation curve model;
adjusting the initial conductive weight value based on the goodness of fit of the initial percolation curve model to make the goodness of fit equal to a best goodness of fit; and
determining the percolation curve model based on a conductive weight value corresponding to the best goodness of fit.

4. The method for determining the material proportion of the conductive material in the battery conductive sheet according to claim 1, wherein the percolation curve model is as follows:

$$y = \alpha(ax_1 + bx_2 + ... + cx_n)^\beta,$$

wherein y is a resistance value of a conductive sheet, $a$ is a conductive weight value corresponding to a first conductive material, $b$ is a conductive weight value corresponding to a second conductive material, $c$ is a conductive weight value corresponding to an n-th conductive material, $\alpha$ is a first fitting parameter, $\beta$ is a second fitting parameter, $x_1$ is a material proportion of the first conductive material, $x_2$ is a material proportion of the second conductive material, and $x_n$ is a material proportion of the n-th conductive material.

5. The method for determining the material proportion of the conductive material in the battery conductive sheet according to claim 1, wherein the preset resistance value of the preset conductive sheet comprises a first sheet resistance value after coating or a second sheet resistance value after cold pressing.

6. The method for determining the material proportion of the conductive material in the battery conductive sheet according to claim 2, wherein the conductive material comprises at least one of a single-walled carbon nanotube conductive material, a multi-walled carbon nanotube conductive material, or a carbon black conductive material, wherein

a material proportion of the single-walled carbon nanotube conductive material is 0 to 0.15;
a material proportion of the multi-walled carbon nanotube conductive material is 0 to 1.5; and
a material proportion of the carbon black conductive material is 0 to 2.

7. An electronic device, comprising:

at least one processor; and
a memory communicatively connected to the at least one processor;
wherein the memory stores a computer program executable by the at least one processor, and the computer program is executed by the at least one processor to enable the at least one processor to perform the method for determining the material proportion of the conductive material in the battery conductive sheet according to any one of claims 1 to 6.

8. A computer-readable storage medium storing computer instructions which, when executed by a processor, cause the processor to perform the method for determining the material proportion of the conductive material in the battery conductive sheet according to any one of claims 1 to 6.

9. An apparatus for determining a material proportion of a conductive material in a battery conductive sheet, comprising:

a percolation curve determination module configured to determine a percolation curve model of a preset conductive sheet, wherein the preset conductive sheet comprises at least one conductive material, and the percolation curve model is a relation curve between a preset resistance value of the preset conductive sheet and a material proportion of each conductive material of the at least one conductive material in the preset conductive sheet; and
a material proportion determination module configured to determine a material proportion of each conductive material in a target conductive sheet based on a target resistance value of the target conductive sheet and the percolation curve model.

Determine a percolation curve model of a preset conductive sheet, where the preset conductive sheet includes at least one conductive material, and the percolation curve model is a relation curve between the preset resistance value of the preset conductive sheet and the material proportion of each conductive material in the preset conductive sheet

S110

Determine the material proportion of each conductive material in a target conductive sheet based on the target resistance value of the target conductive sheet and the percolation curve model

S120

**FIG. 1**

Acquire multiple conductive agent formulations and determine the preset resistance value of a preset conductive sheet corresponding to each conductive agent formulation based on each conductive agent formulation

210

Set the initial conductive weight value of each conductive material and determine an initial percolation curve model based on the initial conductive weight value of each conductive material, the material proportion of each conductive material in each conductive agent formulation, and the preset resistance value of the preset conductive sheet corresponding to each conductive agent formulation

220

Adjust the initial conductive weight value of each conductive material in the initial percolation curve model to determine the percolation curve model

230

Determine the material proportion of each conductive material in a target conductive sheet based on the target resistance value of the target conductive sheet and the percolation curve model

240

**FIG. 2**

## Coated conductive percolation curve model

$$y = 12.5897(22.8x_{swcnt} + 3.75x_{mwcnt} + x_{sp})^{-2.1350}$$
$$R^2 = 0.996792$$

**FIG. 3**

## Cold-pressed conductive percolation curve model

$$y = 11.9899(11.7x_{swcnt} + 1.8x_{mwcnt} + x_{sp})^{-2.2692}$$
$$R^2 = 0.992556$$

**FIG. 4**

FIG. 5

FIG. 6

## INTERNATIONAL SEARCH REPORT

| International application No. |
| --- |
| **PCT/CN2025/079025** |

**A.   CLASSIFICATION OF SUBJECT MATTER**

G16C 60/00(2019.01)i;  G16C 20/20(2019.01)i;  G06F 30/28(2020.01)i;  H01M 4/133(2010.01)i

According to International Patent Classification (IPC) or to both national classification and IPC

**B.   FIELDS SEARCHED**

Minimum documentation searched (classification system followed by classification symbols)

IPC：G16C G06F H01M

Documentation searched other than minimum documentation to the extent that such documents are included in the fields searched

Electronic data base consulted during the international search (name of data base and, where practicable, search terms used)

CNABS; CNTXT; CNKI; WPABS; DWPI; EPTXT; USTXT; WOTXT: 电池, 导电, 膜片, 材料, 占比, 渗流, 曲线, 电阻, battery, conductive, diaphragm, material, proportion, seepage, curve, resistance

**C.   DOCUMENTS CONSIDERED TO BE RELEVANT**

| Category* | Citation of document, with indication, where appropriate, of the relevant passages | Relevant to claim No. |
| --- | --- | --- |
| PX | CN 118538333 A (EVE ENERGY CO., LTD.) 23 August 2024 (2024-08-23)<br>claims 1-8, and description, paragraphs [0004]-[0119] | 1-9 |
| Y | CN 110018348 A (ZHONGTIAN ENERGY STORAGE TECHNOLOGY CO., LTD. et al.)<br>16 July 2019 (2019-07-16)<br>description, paragraphs [0005]-[0136] | 1-9 |
| Y | CN 116027218 A (BYD CO., LTD.) 28 April 2023 (2023-04-28)<br>description, paragraphs [0004]-[0106] | 1-9 |
| A | US 2016323998 A1 (FAMESON TECHNOLOGY CO., LTD.) 03 November 2016<br>(2016-11-03)<br>entire document | 1-9 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 April 2025** | **24 April 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2025/079025**

| Patent document cited in search report | | | Publication date (day/month/year) | Patent family member(s) | | | Publication date (day/month/year) |
|---|---|---|---|---|---|---|---|
| CN | 118538333 | A | 23 August 2024 | None | | | |
| CN | 110018348 | A | 16 July 2019 | CN | 110018348 | B | 20 July 2021 |
| CN | 116027218 | A | 28 April 2023 | CN | 116027218 | B | 11 October 2024 |
| US | 2016323998 | A1 | 03 November 2016 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410592927